# EUROPÄISCHE PATENTANMELDUNG

(11) **EP 4 802 895 A1**
(43) Veröffentlichungstag der Anmeldung: **09.09.2026**
(21) Anmeldenummer: 26162047.0
(22) Anmeldetag: 03.03.2026
(51) Int. Cl.: A01N 43/16, A01P 1/00, A61K 8/368, A61K 8/44, A61Q 17/00, C08L 5/08, C08L 5/10, C11D 1/94

(54) **WÄSSRIGE MITTEL ZUR DESINFEKTION, DEREN HERSTELLUNG SOWIE ANWENDUNGEN**

(30) Priorität: 04.03.2025 DE 202025000779 U
(71) Anmelder: Hartmann, Christoph, 82229 Seefeld (DE); Höglinger, Gerald, 4840 Voecklabruck (AT); Atlantichem GmbH, 82229 Seefeld (DE)
(72) Erfinder: Hartmann, Christoph, 82229 Seefeld (DE)

(57) **Zusammenfassung**

Die vorliegende Erfindung betrifft eine wässrige Zusammensetzung für Desinfektionsanwendungen mit einem a) Polysaccharidsäuretensidkomplex, b) einem Reaktionsprodukt von amphoteren Tensid und einer Carbonsäure, die c) mit einer Carbonsäure auf einen pH-Wert von 1,5 bis 2,5 eingestellt ist, und d) Wasser mit einem Gewichtsanteil von mindestens 92% enthält und frei von stark biozid wirksamen Substanzen wie Aldehyden, Peroxyverbindungen, halogenhaltigen Verbindungen, Phenolen, quartären Ammoniumsalzen, Guanidin und Guanidinderivaten wie Biguanid und Polyhexanid, Übergangsmetallkomplexen und/oder aromatischen Mono- und Polyalkohole ist, sowie deren Herstellung und deren Anwendung als Desinfektionsmittel für Hände, Human- oder Säugetierkörperteile und Human- oder Säugetiergewebe oder für Oberflächen aller Art, textile Gewebe etc.

## Beschreibung

Die vorliegende Erfindung betrifft wässrige Desinfektionsmittel, die gänzlich ohne Alkohol formuliert sind sowie Verfahren zu deren Herstellung und deren Anwendungen.

Die Desinfektion ist eine Hygienemaßnahme, die dazu dient, Krankheitserreger abzutöten bzw. zu inaktivieren und dadurch ihre Anzahl auf oder in einem Objekt bzw. auf einer biologischen Oberfläche deutlich zu reduzieren. Angestrebt wird dabei ein Zustand, in dem eine Infektion nicht mehr wahrscheinlich ist.

Das Deutsche Arzneibuch (DAB) definiert die Desinfektion wie folgt: "totes oder lebendes Material in einen Zustand versetzen, dass es nicht mehr infizieren kann".

Geprüfte Desinfektionsmittel und -verfahren werden in offiziellen Listen aufgeführt, in denen diese nach verschiedenen Einsatzbereichen beschrieben sind: Hygienische und chirurgische Händedesinfektion, Hautantiseptik, Flächen-, Instrumenten-, Wäsche- und Raumdesinfektion sowie Desinfektion von Abfällen. Diese Maßnahmen werden auch als Teil einer Basishygiene bezeichnet.

Desinfektionsmittel sind chemische Substanzen zur Flächen-, Instrumenten- oder Hautdesinfektion sowie zur Wasserentkeimung. Je nach Krankheitserreger (z. B. Bakterien, Viren, Pilzen, Sporen) spricht man diesbezüglich von Bakteriziden, Viruziden (siehe auch Virusinaktivierung), Fungiziden und Sporiziden (siehe auch Biozid und antimikrobielle Substanzen). Sie sind zu unterscheiden von Arzneimitteln wie Antibiotika, Virustatika und Antimykotika, auch wenn diese teilweise ebenfalls oberflächlich eingesetzt werden können.

Zur Desinfektion können chemische oder physikalische Verfahren angewendet werden. Zu den physikalischen Verfahren gehören die Behandlung mit UV-Strahlung oder eine thermische Behandlung durch Hitzeeinwirkung.

Bei der thermischen Desinfektion können sowohl trockene als auch feuchte Hitze zum Einsatz kommen. Die Zellstrukturen von infektiösen Mikroorganismen können dieser thermischen Belastung nicht standhalten und sterben ab.

Bei der Behandlung mit UV-Strahlung, insbesondere UVC-Strahlung, handelt es sich um eine sehr energiereiche elektromagnetische Wechselwirkung mit den Mikroorganismen, die ebenfalls zum Absterben dieser Mikroorganismen führt. Wegen ihres Gefahrenpotentials für Mensch und Tier ist diese Methode jedoch nur für die Desinfektion von Räumen, Oberflächen und Instrumenten geeignet.

Bei den chemischen Verfahren werden Substanzen eingesetzt, die nachweislich eine entsprechende Wirksamkeit gegenüber den Infektionsquellen aufweisen.

Sehr häufig eingesetzte Wirkstoffe sind Alkohole wie Ethanol, 1-Propanol, Isopropanol/IPA oder Aldehyde wie Glutaraldehyd und Formaldehyd.

Die Wirkungsweise von Alkoholen genauso wie die von Aldehyden beruht auf der Denaturierung von Proteinen, was zum Absterben der Mikroorganismen führt.

Weitere im Einsatz befindliche Desinfektionsmittel sind starke Oxidationsmittel wie z.B. halogenhaltige Produkte wie Chlor, Chlorhexidin, Chlordioxid, Natriumhypochlorit, Ozon oder Jod. Starke Oxidationsmittel auf Basis Aktivsauerstoff sind Wasserstoffperoxid sowie organische und anorganische Abkömmlinge von Wasserstoffperoxid wie Peressigsäure oder Caroat, ein Mischsalz der Monoperoxyschwefelsäure.

Eine andere Möglichkeit pathogene Keime zu bekämpfen, besteht in der Methode einer Enzymhemmung wie sie von bestimmten Metallkomplexen geleistet wird. Besonders Verbindungen des Kupfers, Silber und Quecksilber sind hier zu nennen.

Weitere Gruppen von Desinfektionsmittel umfassen die Substanzklassen der phenolischen Verbindungen wie z.B. Kresole, Chlorxylenol, Triclosan und quartäre Ammoniumverbindungen, sogenannte Quats wie z.B. Benzalkoniumchlorid. Unter den stickstoffhaltigen Desinfektionsmitteln sind vor allem zu nennen Guanidin und Derivate davon sowie Octinidin und Polyhexanid.

All diese Substanzen erfüllen ihren Zweck, nämlich gefährliche mikrobiologische Organismen so weit zu reduzieren oder abzutöten, daß sie keine bedrohlichen Infektionen mehr auslösen können.

Allerdings haben viele dieser genannten antimikrobiell wirksamen Substanzen auch Nebenwirkungen, die in Kauf genommen werden müssen. So haben all diese genannten Substanzgruppen von wirksamen chemischen Desinfektionsmitteln eine Reihe von Nachteilen, die man in Kauf nehmen muß. So z.B. daß im Falle einer Flächendesinfektion die Oberflächen angegriffen und geschädigt werden können. Bei Desinfektion im humanen Bereich, z.B. der alltäglichen Handdesinfektion können durch den Hautkontakt Allergien ausgelöst werden. Ein weiterer unangenehmer Effekt bei, der weitaus am häufigsten angewendeten Methode der Handdesinfektion mit alkoholischen Desinfektionsmitteln ist, daß der permanente Kontakt der Hand mit Alkohol zur Aufrauhung der Haut führt. Der Alkohol löst nämlich die wichtigen Hautfette und führt zur Austrocknung der Haut, speziell der Handflächen, was sich häufig durch unangenehme Rißbildungen der Haut zeigen kann.

Es besteht also Bedarf nach Alternativen, die diese Nebenwirkungen nicht mehr vorweisen aber dennoch den Anforderungen für eine zuverlässige Desinfektion vollauf genügen.

Insbesondere bei der Flächen- und Handdesinfektion wäre es wünschenswert Desinfektionsmittel zum Einsatz zu bringen, die die Oberflächen nicht angreifen und vor allem bei der Handdesinfektion die Haut nicht austrocknen lassen und kein Potential haben, Allergien auslösen zu können.

So haben die mit Abstand am häufigsten eingesetzte Handdesinfektionsmittel einen sehr hohen Gehalt an Alkohol wie Ethanol, 1-Propanol, Isopropanol oder Mischungen davon. Dies führt bei dauerhaftem Einsatz, wie es für das medizinische und Pflegepersonal die Regel ist, zu unangenehmen Austrocknen der Haut, da der Alkohol als sehr gutes Lösungsmittel die Hautfette allmählich herauslöst und die Hände oder Haut dadurch sehr stark austrocknen und rissig werden.

Speziell für diese Anwendung besteht also ein starkes Bedürfnis Alternativen zu finden, die ohne Alkohol, Aldehyde, starke Oxidationsmittel, Phenole und quartäre Ammoniumsalze auskommen. Quartäre Ammoniumverbindungen, gelegentlich auch QAV oder Quats genannt, sind organische Ammonium-Verbindungen, bei denen an alle vier Valenzen des Stickstoffatoms organische Reste gebunden sind.

Eine besondere Herausforderung für die angesprochenen Flächen- und Handdesinfektionsmittel stellen die Zielanforderungen für diese Anwendungen dar. Für die Desinfektion von Flächen und Händen müssen die Desinfektionsmittel, im Gegensatz zu Konservierungsmitteln, in kurzen Zeitabschnitten die Inaktivierung von mikrobiellen Keimen sicherstellen. Hinzu kommt die Forderung, daß diese Mittel ein sehr breites Spektrum an Keimen zu bekämpfen haben.

Neben diesen anspruchsvollen Aufgaben muß das Desinfektionsmittel zudem toxikologisch völlig unbedenklich, hypoallergen und idealerweise ökologisch kompatibel sein.

Schon lange ist bekannt, daß bestimmte Biopolymere über eine mikrobielle Wirksamkeit verfügen. Diese Substanzen sind schon deshalb besonders interessant, da sie optimale biologische Kompatibilität zeigen, ökologisch sinnvoll und unbedenklich und auch entsprechend verfügbar sind. Anwendungen dieser Biopolymere folgen dem biomimetischen Ansatz, d.h. man versucht die Natur als Vorbild zu nehmen und dies dann umzusetzen in entsprechende Produktlösungen.

Hierbei besonders hervorstechende und weitverbreitete Biopolymere sind stickstoffhaltige Polysaccharide und insbesondere das Polyglucosamin Chitosan, das aus dem zweithäufigsten Naturstoff, dem Chitin gewonnen werden kann.

Chitin ist ein Polysaccharid, das aus Acetylglucosamin-Einheiten aufgebaut ist (präzise: 2-Acetamido-2-desoxy-D-glucopyranose, kurz: N-Acetyl-D-glucosamin). Die Acetylglucosamin-Einheiten sind durch β-1,4-glycosidische Bindungen verknüpft - dies ist die gleiche Bindungsart wie die der Glucosemoleküle in Cellulose. Chitin kann also als Abart der Cellulose aufgefasst werden, bei der die Hydroxygruppen in Position 2 der Monomereinheiten durch Acetamidogruppen ersetzt wurden.

Das Chitosan wird technisch aus Chitin (Poly (N-Acetyl-1,4-β-D-Glucopyranosamin) durch Deacetylierung gewonnen, so dass das Molekül schließlich überwiegend nur noch aus linear miteinander verbundenen 2-Amino-2-desoxy-β-D-glucopyranose- bzw. Glucosamin-Monomeren besteht. Die Deacetylierung kann entweder klassisch chemisch durch eine Art Verseifungsreaktion durch Behandlung mit starken Laugen erfolgen. Neuerdings zeichnet sich jedoch ein alternativer und sehr eleganter Weg der Deacetylierung ab. Hierbei wird die Acetylgruppe enzymatisch abgespalten, also ohne "harte"und nicht besonders umweltfreundliche Chemie einzusetzen. Der Prozentsatz der deacetylierten Acetylglucosamin-Einheiten gibt den Deacetylierungsgrad, einer wichtigen Kenngröße des Chitosans, an.

Schließlich gibt es noch die Möglichkeit das Chitosan direkt aus selektiv gezüchteten Pilzen herzustellen. Diese Variante hat zudem den Vorteil, daß das so erhaltene Chitosan veganer Natur ist, also frei jeglichen tierischen Ursprungs ist.

Chitosan ist ein farbloser, amorpher, hochmolekularer Feststoff, der in Wasser nahezu unlöslich und in verdünnter Salzsäure sowie in vielen organischen Säuren löslich ist.

Die Löslichkeit in Säuren und gleichzeitig schlechte Löslichkeit im neutralen oder alkalischen pH-Bereich ist einzigartig unter den Biopolymeren und daher charakterisierend.

Die Besonderheit des Chitosans liegt vor allem in der Anwesenheit von freien Aminogruppen, die durch die Deacetylierung des Chitins entstanden sind. Diese Aminogruppen werden dann durch die Säuren protoniert und in Ammoniumgruppen überführt. Dann liegt das Chitosan als Polykation vor was zum einen die Wasserlöslichkeit und zum anderen auch die antimikrobiellen Eigenschaften erklären kann.

Die antimikrobiellen Eigenschaften des Chitosan's sind schon sehr lange bekannt und daher wurden schon sehr früh Chitosanpräparate für den Einsatz im medizinischen Sektor entwickelt und angewendet. Insbesondere bei der Behandlung von Wunden haben sich derartige Produkte erfolgreich einsetzen lassen. Hier zeigen Chitosanpräparate ganz ausgezeichnete Eigenschaften, da sie nicht nur wegen ihrer antimkrobiellen Wirkung eine ständige Neuinfektion der Wunden verhindern, sondern durch ihre ausgezeichnete Fähigkeit sehr dünne, Sauerstoff durchlässige Filme zu bilden, die Verschließung offener Wunden beschleunigen.

Ein besonderer Vorzug Chitosan haltiger Präparate beruht jedoch auf der Tatsache, daß Chitosan humantoxikologisch völlig unbedenklich ist und hypoallergene Eigenschaften besitzt.

Es gab daher eine ganze Reihe von Versuchen, Chitosan basierte Produkte im Bereich der antimikrobiellen Behandlung, insbesondere im wichtigen Segment der Desinfektion einzusetzen.

In der CN 104622710 A wird eine Kombination von Salicylsäure mit Chitosan beschrieben, mit einem Gewichtsverhältnis von Salicylsäure zu Chitosan von 1:1 bis 1:1,8. Diese Kombination enthält außerdem alkoholische Substanzen wie z.B. Ethanol oder Glycerin und findet Anwendung auf dem Gebiet der Kosmetik.

Auch in der US 2008/0045491 A1 wird eine Kombination von Salicylsäure, Chitosan und einem wasserlöslichen Alkohol ein Mittel beschrieben, das sowohl zur Händedesinfektion als auch zur Flächendesinfektion eingesetzt werden kann.

Eine antimikrobiell wirksame Mischung aus Chitosan, Salicylsäure und Polyhexanid (Polyhexamethylen biguanid (PHMB)) einem antimikrobiellen Wirkstoff, wird in der US 2007/0048356 A1 beschrieben. Dieses Mittel dient zum Coaten von Oberflächen, um eine antimikrobielle Wirkung zu erzielen.

Für eine Anwendung in sogenannten Feuchttüchern zur Hautpflege wird eine Kombination von Salicylsäure mit Chitosan in der WO 02/49604 A1 beschrieben. Dieses Mittel soll als Konservierungsmittel eingesetzt werden und dadurch das Verkeimen solcher Feuchttücher verhindern.

In der EP 4 088 784 ist eine wässrige Dreierkombination bestehend aus einem Polysaccharid, das aus Aminozuckereinheiten aufgebaut ist, z.B. Chitosan, einer Carbonsäure, z.B. Mandelsäure, Gallussäure, Salicylsäure, 4-Hydroxybenzoesäure, Bernsteinsäure, Phthalsäure, Trimesinsäure und deren Mischungen und einem als Biozid bekanntem antimikrobiellen Wirkstoff wie z.B. einem aliphatischen Polyamin und/oder einem aliphatischen Guanid beschrieben. Diese Mittel können für kosmetische als auch für Desinfektionszwecke eingesetzt werden.

In der US 2003/0092597 A1 wird ein wässriges Reinigungsmittel für harte Oberflächen beschrieben, das neben Tensiden, organischen Säuren auch ein Poly-D-glucosamin wie z.B. Chitosan enthält. Allerdings ist hier die Anwendung auf Reinigung von harten nicht flexiblen Oberflächen wie in Küchen, Bädern, Duschen oder Toiletten beschränkt. Eine Anwendung als Hände- und Flächendesinfektionsmittel ist nicht vorgesehen.

In einem Sicherheitsdatenblatt für ein Produkt BPC 229 der BPC Specialties GmbH: MSDS Version 01.12.Januar 2021 https://bpc.bio/coa-m229/ wird als aktiver Bestandteil in Kosmetik- Hygiene- und Färbeprodukte ein Substanzgemisch bestehend aus Wasser, Betainsalicylat und Cocoamidopropyl Betain genannt. Jedoch ist hier der Anteil des aktiven Wirkstoffs bei 13 % w/w angegeben und damit sehr hoch. Zudem liegt der pH-Wert dieser Mischung bei 3,5 - 4,5 und unterscheidet sich damit signifikant vom pH-Wert der vorliegenden Erfindung, welcher bei 1,7 - 2,2 liegt.

Die EP 0 649 437 B1 beschreibt einen Charge-Transfer-Komplex von lod mit Chitosan und Derivaten davon sowie ein Verfahren zu dessen Herstellung. Diese wasserlöslichen Komplexverbindungen machen eine retardierte Freigabe von Jod möglich und können somit als desinfizierende Wundbehandlungsmittel eingesetzt werden.

Die EP 4 356 733 A1 beschreibt ein niedrigalkoholisches Desinfektionsmittel zur Behandlung von unbelebten Oberflächen, das eine Kombination von maximal 50% eines einwertigen Alkohols und weniger als 1% einer organischen Dicarbonsäure und einer organischen Sulfonsäure, mit einem pKs-Wert von kleiner 2,3 darstellt.

Die aus dem Stand der Technik alkoholfreien bekannten Beispiele erfüllen jedoch nicht die zum Ziel gesetzten Bedingungen, nämlich, daß das Mittel schnell genug seine antimikrobielle Wirksamkeit entfaltet, so daß es einem Alkohol basierten Desinfektionsmittel nicht unterlegen ist und/oder es enthält Substanzen, die weiterhin zu Unverträglichkeiten mit den behandelten Oberflächen oder der menschlichen Haut führen kann.

Es besteht also weiterhin die Aufgabenstellung, ein wässriges, alkoholfreies Desinfektionsmittel bereitzustellen, das einen behördlich vorgeschriebenen Test zur Hand- und Flächendesinfektion bestehen kann, damit es auch als geprüft wirksames Desinfektionsmittel in medizinischen Einrichtungen sowie Pflegeeinrichtungen problemlos eingesetzt werden kann und das auf Zusatz von bekannten und stark antimikrobielle wirkende Substanzen verzichten kann und auf Basis von Biopolymeren in Kombination mit Tensiden und organischen Säuren und deren Derivaten aufgebaut ist.

Gegenstand der vorliegenden Erfindung ist daher eine wässrige Lösung für die Anwendung in der Desinfektion von Oberflächen von Körperteilen, Händen und humanem oder Säugetiergeweben sowie Oberflächen von Gegenständen, Räumen, Geräten und Kleidung ohne Zusatz von Alkohol oder bekannte, stark antimikrobiell wirkende Substanzen.

Im Rahmen der vorliegenden Erfindung konnte gezeigt werden, daß überraschenderweise die Kombination von Chitosan-Säure-Tensid-Trikomplexen mit Glycinbetainsalicylat und anschließender Optimierung durch pH-Einstellung der wässrigen Lösung mit organischen Säuren in niedrigen Konzentrationen zu dem gewünschten Ergebnis geführt hat. Die so erhaltene wässrige Lösung zeigt eine signifikante Verstärkung der antimikrobiellen Wirksamkeit gegenüber den einzelnen Komponenten der wässrigen Lösung, ohne Zusatz von bekannten, stark antimikrobiell wirkenden Substanzen und ohne daß eine hohe Konzentration an freier organischer Säure eingesetzt werden muß, was zu Hautirritationen führt.

Wie die mikrobiologischen Labortests zeigen, handelt es sich hierbei um einen echten synergistischen Effekt, da die Summe der Keimzahlreduktionen der einzelnen Komponenten, also Chitosankomplex, Glycinbetainsalicylat und pH-Einstellung kleiner ist als die Keimzahlreduzierung des erfindungsgemäßen wässrigen Desinfektionsmittels. Zudem ist es höchsterstaunlich und überraschend, daß die Zusammensetzung des Mittels, welches Gegenstand dieser Erfindung ist, in sehr kleinen Konzentrationen an Inhaltsstoffen und damit verbunden einem sehr hohen Wassergehalt, sehr schnell wirksame und langanhaltende mikrobiologische Aktivität, also Reduktion eines sehr breiten Mikrobenspektrums, aufweist. Diese enorme mikrobiologische Effizienzsteigerung ist das Resultat des synergistischen Zusammenwirkens der drei einzelnen Komponenten des erfindungsgemäßen Mittels.

Die in dieser Erfindung eingesetzten Chitosankomplexe bestehend aus Chitosan, organischen Säuren und Tensiden sind prinzipiell bekannt und z.B. in der DE 10 2021 109 834 A1 aufgeführt. Allerdings werden dort diese Komplexe noch mit handelsüblichen Bioziden kombiniert, z.B. mit Polyaminen und insbesondere mit Guaniden wie Biguanid, um deren Wirksamkeit so zu erhöhen, daß sie für die Anwendung als Flächen- und Handdesinfektionsmittel geeignet sind.

Unsere Aufgabenstellung hingegen war es auf die Verwendung von stark biozid wirkenden Agentien wie Alkohole, quartäre Ammoniumsalze (QUATS), Guanidin und Guanidinderivate etc. in unserem erfindungsgemäßen rein wässrigen Desinfektionsmittel ganz zu verzichten.

Überraschenderweise wurde gefunden, daß die Kombination dieser Chitosankomplexe mit dem in der Kosmetik gerne eingesetzten Glycinbetainsalicylat,(CAS Nr. 17671-53-3), welches selbst nur schwach gegen Mikroorganismen wirkt, mikrobiologische Eigenschaften aufweist, und einer genauen pH-Einstellung mit einer Carbonsäure zu einer deutlichen antimikrobiellen Wirkungssteigerung führt.

Die Strukturanalysen des Glycinbetainsalicylats belegen, daß es sich hier nicht um ein klassisches quartäres Ammoniumsalz handelt, sondern um ein Addukt der beiden Komponenten. Diese Verbindung wird daher der Gruppe der Cocrystals zugerechnet, bei der durch starke Wasserstoffbrückenbindungen die individuellen Moleküleigenschaften der beiden Einzelmolekülen, also Glycinbetain und Salicylsäure signifikant erhalten bleiben, was bei einem quartären Ammoniumsalz nicht der Fall ist.

Ein weiterer Bestandteil der Erfindung ist es, daß die wässrige Lösung des Chitosan-Säure-Tensid-Komplexes und dem Glycinbetainsalicylat gerade mit so viel an organischer Säure, wie z.B. Zitronensäure versetzt wird, damit das fertige erfindungsgemäße Produkt einen pH-Wert im Bereich von 1,8 bis 2,2, vorzugsweise einen pH-Wert von 1,9 hat.

Während der Chitosantrikomplex wie im Ausführungsbeispiel gezeigt hergestellt werden muß, ist die Komponente Glycinbetainsalicylat kommerziell leicht erhältlich und wird gerne in Kosmetikprodukten eingesetzt.

Da alle in dem erfindungsgemäßen Desinfektionsmittel zum Einsatz kommenden Substanzen in den weiter unten konkretisierten Konzentrationsbereichen humantoxikologisch als völlig unbedenklich zu bewerten sind, ist auch das der Erfindung zugrundeliegende Desinfektionsmittel trotz seiner schnellen und hocheffizienten mikrobiologischen Wirksamkeit als völlig unbedenklich und absolut hautverträglich einzustufen und eine entsprechende Kennzeichnung der Verpackung mit Gefahrensymbolen entfällt aus diesem Grunde komplett.

Bei der Erfindung handelt es sich somit um ein hochwirksames Desinfektionsmittel, das völlig ohne Zusatz von stark biozid wirkenden Substanzen, wie z.B. Alkohole oder den weiter oben bezeichneten, human- und ökotoxikologisch nicht immer unbedenklichen Desinfektionsmittelwirkstoffen auskommt und der Gehalt an Wasser, als einziges Lösungsmittel, größer 92% ist. Dieser sehr hohe Wassergehalt unter den gerade erwähnten Parametern der Inhaltsstoffe ist ungewöhnlich und erneut nur auf die synergistische Wirkungsweise der Komponenten dieser Zusammensetzung des erfindungsgemäßen Mittels zurückzuführen.

Schließlich ist es Bestandteil dieser Erfindung, daß das Mittel zur Anwendung im Bereich der Desinfektion von Oberflächen von Körperteilen, Händen und humanem oder Säugetiergeweben sowie Oberflächen von Gegenständen, Räumen, Geräten und Kleidung, ohne Einschränkung was die Materialbeschaffenheit der Oberflächen anbelangt, eingesetzt werden kann.

Wegen seiner filmbildenden Eigenschaften sowohl des Chitosan Komplexes als auch des Glycinbetainsalicylats sorgt ein hauchdünner Film der mit dem Mittel, welches Gegenstand dieser Erfindung ist, behandelten Oberflächen für eine antimikrobielle Ausrüstung der behandelten Oberflächen mit andauernder Schutzwirkung, zumindest so lange, bis dieser Film durch Kontakt mit Wasser wieder ausgewaschen wird.

Zum Nachweis dieses synergistischen Effektes wurden mikrobiologische Test durchgeführt, bei denen die Entwicklung von keimbildenden Einheiten auf Agartestplatten ausgezählt wurden. Hierzu werden Agarplatten mit geeigneten und einer Auswahl, siehe unten, von bestimmten Keimen mit dem Nährmedien ausplattiert. Die Bebrütung erfolgt bei 38°C und nach jeweils drei Inkubationszeiten können die Kolonien auf den Platten ausgezählt und somit die Keimzahlen bestimmt werden. Die Angabe erfolgt in KBE/ml, was so viel bedeutet wie Kolonienbildende Einheiten pro Milliliter Probe.

Eingesetzte Mikrobenstämme:
Citrobacter amalonaticus, Citrobacter freundii, Listeria monocytogenes, Pseudomonas protegens, Citrobacter braakii, Achromobacter denitrificans
Es werden die KBE-Werte von einer unbehandelten Probe mit den Proben verglichen, die jeweils mit 0,5ml der verschiedenen Komponenten versetzt wurden. Die Komponenten, die zugesetzt wurden, um die mikrobiologische Wirksamkeit festzustellen wurden jeweils als Lösung in den Konzentrationen zugegeben, welche dem erfindungsgemäßen Produkt entspricht.

**Tabelle 1 : Ergebnisse der Vergleichsversuche Keimzahlen mit unterschiedlicher Beimpfung**

| Komponente | KBE nach 24 Std. | KBE nach 48 Std. | KBE nach 72 Std. |
|---|---|---|---|
| Blindprobe, ohne Zusatz | überwachsen | überwachsen | überwachsen |
| Chitosansalicylsärecocoamidopropylbeta inkomplex 2,6% ig | 12 | überwachsen | überwachsen |
| Glycinbetainsalicylat 0,4% ig | 4 | 9 | überwachsen |
| Chitosansalicylsärecocoamidopropylbeta inkomplex + Glycinbetainsalicylat 3% ig, pH = 3,85 | 12 | überwachsen | überwachsen |
| Zitronensäure wässrige Lösung 3,95% ig | 4 | 8 | 270 |
| Erfindungsgemäßes Produkt 6,95% ig, pH = 1,9 | 0 | 0 | 0 |

Wie oben gezeigt bleibt festzuhalten, daß die Einzelkomponenten der Erfindung jeweils für sich nicht die in den Ansprüchen dieser Erfindung festgelegten Wirkung aufweisen können. Erst die Kombination der Einzelkomponenten in der erfindungsgemäßen Zusammensetzung des Mittels können diesen synergistischen Effekt vorweisen und zu den ebenfalls beanspruchten Anwendungen als Flächen- und Handdesinfektionsmittel eingesetzt werden.

Das der Erfindung zugrunde liegende Mittel zur Desinfektion setzt sich wie folgt zusammen:

### a) einem Polysaccharid-Säure-Tensid-Komplex aus:

(A) einem Polysaccharid bestehend aus Aminozuckereinheiten, insbesondere Glucosaminen und oder Galactosaminen, (B) einer Carbonsäure, bevorzugt einer Hydroxycarbonsäure wie die Fruchtsäuren, besonders bevorzugt einer aromatischen beta-Hydroxycarbonsäure wie Salicylsäure und (C) einem Tensid, bevorzugt einem nichtionischen oder amphoteren Tensid und besonders bevorzugt dem Glycinbetain also N, N, N-Trimethylammonioacetat und/oder dem Cocoamidopropylbetain (CAPB).

Die Polysaccharide (A) welche bevorzugt sind, sind das Chitosan und Chitosanderivate. Bei den Chitosanderivaten sind besonders N-Carboxymethylchitosan, Hydroxypropylchitosan und N, N, N-Trimethylchitosan bevorzugt.

Für die vorliegende Erfindung kommen sehr viele Carbonsäuren (B) infrage. Besonders bewährt haben sich alpha- und beta-Hydroxycarbonsäuren, insbesondere die Salicylsäure und Polysäuren, insbesondere Di- und Tricarbonsäuren, wie die Fruchtsäuren Wein-, Äpfel-, Milch-, Mandel- und Zitronensäure sowie Glycolsäure. Es können auch Mischungen der genannten Carbonsäuren zum Einsatz kommen.

Um die Löslichkeit, Dispergierfähigkeit des Chitosan-Carbonsäure-Komplexes in Wasser sowie die gleichmäßige Verteilung auf der zu behandelnden, sprich zu desinfizierende Oberfläche für eine bessere Filmbildung zu erhöhen, werden optional noch bestimmte Tenside (C) in den Chitosan-Säure-Komplex integriert. Um die Struktur des Chitosan-Carbonsäure-Komplexes dabei nicht zu stören, hat es sich als vorteilhaft erwiesen, daß man die Tenside aus der Gruppe der nichtionischen oder amphoteren Tenside wählt. Besonders geeignet für die Herstellung des erfindungsgemäßen Mittels sind bei den amphoteren Tensiden Betaine wie z.B. das Glycinbetain oder das Cocoamidopropylbetain. Bei den nichtionischen Tensiden sind Vertreter der Gruppe der Alkylpolyglycoside (APGs) bevorzugt.

### b) einer Betain-Salicylat Komponente:

Es ist weiterhin essenzieller Bestandteil dieser Erfindung, nach der Aufnahme des Chitosan-Carbonsäure-Tensid-Komplexes a) in Wasser diese Lösung mit einem Betainsalicylat zu versetzen. Das bevorzugte Betainsalicylat, das (Carboxymethyl)trimethylammonium salicylat; (CAS Nr. 17671-53-3) ist ein in der Kosmetik häufig eingesetztes Produkt, bei der es sich um ein Kombinationsprodukt bestehend aus Glycinbetain,und Salicylsäure handelt. Wie in dem Chitosan-Carbonsäure-Tensid-Komplex so ist auch in diesem Betain-Salicylsäure-Komplex die aggressive Wirkungsweise der freien Salicylsäure durch die Komplexierung mit dem Betain
merklich reduziert. Die Zugabe des Betainsalicylats allein bedingt allerdings noch nicht eine deutliche Steigerung der antimikrobiellen Wirksamkeit der wässrigen Lösung des Chitosan-Carbonsäure-Tensid-Komplexes. Beide Komponenten für sich, also einmal der Chitosan-Carbonsäure-Komplex mit optionalem Tensid und zum anderen das Betainsalicylat sind jeweils und auch additiv nicht stark genug in ihrer antimikrobiellen Wirkung um die strengen Vorgaben z.B. der VAH oder RKI für eine Zulassung als Hand- und Flächendesinfektionsmittel zu bestehen.

### c) pH-Wert Optimierung:

Ein weiterer essenzieller Bestandteil dieser Erfindung ist die abschließende pH-Adjustierung der oben im Allgemeinen beschriebenen wässrigen Lösung, bestehend aus dem ChitosanKomplex und dem Betainsalicylat. Dies geschieht mithilfe von Carbonsäuren wie sie bereits bei der Herstellung des Chitosan-Carbonsäure-Komplexes eingesetzt werden, vorzugsweise Zitronensäure. Es wird dann jeweils so viel einer konzentrierten, also gesättigten wässrigen Lösung dieser Carbonsäuren zugegeben, so daß der pH-Wert des fertigen Desinfektionsmittels im Bereich 1,8 bis 2,2, vorzugsweise bei 1,9 liegt.

Diese pH-Optimierung mit Zitronensäure, ergibt dann eine Konzentration der Zitronensäure von 3,9 Gewichtsprozent im gebrauchsfertigen erfindungsgemäßen Mittel, eine Zitronensäurekonzentration, die für sich allein noch keine ausreichend starke mikrobiologische Aktivität besitzt. Überraschenderweise hat jedoch die Kombination, bestehend aus den beiden Komponenten a) und b) nach anschließender pH-Optimierung c) zu einer deutlichen Steigerung der mikrobiologischen Aktivität des erfindungsgemäßen Mittels geführt, so daß das Mittel die gesetzten Ziele für eine Zulassung als Flächen- und Handdesinfektionsmittel erhalten kann.

Eine besonders bevorzugte Ausführungsform gemäß der vorliegenden Erfindung hat folgende Zusammensetzung.

### (a) Chitosan-Salicylsäure-Cocoamidopropylbetain-Komplex (Trikomplex).

Das eingesetzte Chitosan ist kommerziell erhältlich und weist folgende Spezifikationswerte auf: Deacetylierungsgrad größer 70% und idealerweise mindestens 90% und einem mittleren Molekulargewicht von 30 - 1000 kDa, bevorzugt im Bereich von 50 - 700 kDa, insbesondere von 80 - 200 kDa.

Die Salicylsäure ist als kommerzielles Produkt im Handel erhältlich, ebenso wie das amphotere Tensid Cocoamidopropylbetain.

Der aus den Edukten Chitosan, Salicylsäure und Cocoamidopropylbetain in Wasser hergestellte Chitosan-Trikomplex enthält das Chitosan in einer Gewichtskonzentration von 0,01% bis 0,5%, bevorzugt 0,05% bis 0,2%, besonders bevorzugt von 0,07% bis 0,09%, die Salicylsäure in einer Gewichtskonzentration von 0,01% bis 0,3%, bevorzugt 0,03% bis 0,15%, besonders bevorzugt von 0,05% bis 0,08%, und das Cocoamidopropylbetain in einer Gewichtskonzentration von 1% bis 15%, besonders von 1,5% bis 10% und besonders bevorzugt von 2,0% bis 3,0%, immer bezogen auf das erfindungsgemäße und gebrauchsfertige Produkt.

Die jeweils zu 100% Gewichtsanteile fehlenden Gewichtsprozente sind Wasser, als einziges Lösungsmittel des erfindungsgemäßen Mittels.

Das erfindungsgemäße Mittel als gebrauchsfertiges Produkt enthält dann 0,1% bis 15%, bevorzugt 0,5% bis 10%, besonders von 1% bis 5,0%, besonders bevorzugt 2,0% bis 3,0 % Gewichtsanteile des Chitosan-Trikomplexes (a) bezogen auf das wässrige, gebrauchsfertige Mittel dieser Erfindung.

### (b) Glycinbetain-Salicylat ((Carboxymethyl)trimethylammonium salicylat)

Diese Komponente ist ein kommerziell erhältliches Produkt, das in der Kosmetikindustrie im Einsatz ist. Das Gewichtsmengenverhältnis vom Chitosan-Trikomplex zum Glycinbetain-Salicylat im gebrauchsfertigen, erfindungsgemäßen Produkt liegt bevorzugt im Bereich von 10:1 bis 1:10 und ganz besonders 6,8:1.

Das erfindungsgemäße Mittel als wässriges, gebrauchsfertiges Produkt enthält dann 0,05% bis 5%, bevorzugt 0,1% bis 2,5%, besonders von 0,2% bis 1,0%, besonders bevorzugt von 0,35% bis 0,45% Gewichtsanteile Glycinbetain-Salicylat (b) bezogen auf das wässrige, gebrauchsfertige Mittel dieser Erfindung.

### (c) Carbonsäuren zur pH-Adjustierung

Hier kommen mindestens eine Carbonsäure aus der Gruppe der Hydroxysäuren wie die Glykolsäure, Zitronensäure, Milchsäure, Mandelsäure, Äpfelsäure, Gallussäure, Mecallonsäure, Isozitronensäure, Tatronsäure, beta-Hydroxybuttersäure, 4-Hydroxybenzoesäure, Salicylsäure und deren Mischungen zum Einsatz.

Die in Wasser vorgelösten Carbonsäuren, bevorzugt die Zitronensäure, werden der wässrigen Lösung bestehend aus dem Chitosantrikomplex (a) und dem Glycinbetain-Salicylat (b) so zugegeben, daß die gebrauchsfertige, wässrige Lösung, die Gegenstand dieser Erfindung ist, einen pH-Wert im Bereich von 1,5 bis 2,5, insbesondere 1,7 bis 2,2 und ganz besonders von 1,9 aufweist. Das erfindungsgemäße Mittel als wässriges, gebrauchsfertiges Produkt enthält dann 0,1% bis 10%, bevorzugt von 1% bis 7%, besonders von 3% bis 5% Gewichtsanteile Zitronensäure bezogen auf das wässrige, gebrauchsfertige Mittel dieser Erfindung.

### (d) Wasser als Lösungsmittel

Das wässrige, gebrauchsfertige, erfindungsgemäße Produkt enthält ausschließlich Wasser als Lösungsmittel, in dem die drei oben aufgeführten Rezepturbestandteile in einer klaren Lösung gelöst sind. Der gewichtsmäßige Anteil an Wasser in dem gebrauchsfertigen, erfindungsgemäßen Produkt beträgt mindestens 92%.

Ausführungsbeispiel:
I) Es werden 1,0 g Chitosan (DA 90%; MW 120 kDa) mit 0,8 g Salicylsäure in 100ml Wasser aufgenommen und vermischt. Anschließend werden 900 ml Wasser unter Rühren langsam hinzugefügt und unter kräftigem Rühren bei 80°C so lange weitergerührt, bis sich ein schwach gelbliches Gel gebildet hat.
II) Nun werden 150g einer 20%igen wässrigen Lösung von Cocoamidopropylbetain portionsweise bei 60°C in das Gel eingerührt und so lange gerührt bis sich eine klare, gelartige Lösung gebildet hat.
III) Zu dieser gelartigen Lösung werden nun bei Raumtemperatur 4,7 g Glycinbetainsalicylat eingerührt.
IV) Um schließlich den pH-Wert der so hergestellten Lösung III) auf 1,9 einzustellen, werden 78,8g einer konzentrierten Zitronensäure Lösung (= 1630g Zitronensäure Monohydrat. in 1000g Wasser) in die Lösung III) eingerührt.

Schließlich wird diese Lösung filtriert, um sie von eventuell ausgefallenen Feststoffen zu befreien.

Das so hergestellte Desinfektionsmittel zeigt die antimikrobiellen Wirksamkeiten wie weiter oben in der Tabelle 1 gezeigt.

## Patentansprüche

1. Wässrige Zusammensetzung für Desinfektionsanwendungen enthaltend
a) einen Polysaccharidsäuretensidkomplex bestehend aus
(A) einem Polysaccharid bestehend aus Glucosamin-Monomereinheiten oder Galactosamin-Monomereinheiten, und
(B) einer Carbonsäure, und
(C) einem nichtionischen oder amphoteren Tensid
b) ein Reaktionsprodukt von amphoteren Tensiden mit einer Carbonsäure
und c) einen Mindestgewichtsanteil Wasser von 92 %,
**dadurch gekennzeichnet, dass** diese Zusammensetzung keine Substanzen aus den Stoffgruppen der Aldehyde, Peroxyverbindungen, halogenhaltigen Verbindungen, Phenole, quartären Ammoniumsalzen, kein Guanidin und Guanidinderivaten wie Biguanid und Polyhexanid, Übergangsmetallkomplexe und/oder aromatischen Mono- und Polyalkohole enthält und einen pH-Wert Bereich 1,5-2,5, vorzugsweise von 1,7 bis 2,2 und ganz besonders einen pH-Wert von 1,9 aufweist

2. Wässrige Zusammensetzung nach Anspruch 1, **dadurch gekennzeichnet, daß** das Polysaccharid (A) Chitosan oder ein Chitosanderivat ist,
die Carbonsäure (B) Salicylsäure ist, das nichtionische oder amphotere Tensid (C) Cocoamidopropylbetain ist, und das Reaktionsprodukt von amphoteren Tensiden mit einer Carbonsäure b) ((Carboxymethyl)trimethylammoniumsalicylat) ist.

3. Wässrige Zusammensetzung gemäß Ansprüchen 1 oder 2, **dadurch gekennzeichnet, daß** das Polysaccharid in Mengen von 0,01% bis 0,5%, bevorzugt 0,05% bis 0,2%, besonders von 0,08% bis 0,1%, besonders bevorzugt von 0,09%, die Carbonsäure in Mengen von 0,01% bis 0,5%, bevorzugt 0,03% bis 0,15%, besonders von 0,05% bis 0,09%, besonders bevorzugt von 0,07%, das nichtionische oder amphotere Tensid in Mengen von 1% bis 15%, besonders von 2% bis 10% und bevorzugt von 2,5% bis 5%, besonders bevorzugt von 2,6%, das Reaktionsprodukt von amphoteren Tensiden mit einer Carbonsäure in Mengen von 0,05% bis 5%, bevorzugt 0,1% bis 2,5%, besonders von 0,2% bis 1,0%, besonders bevorzugt von 0,4%, Wasser in Mengen von mindestens 92% bezogen auf die Masse der Zusammensetzung enthalten sind.

4. Wässrige Zusammensetzung nach einem der Ansprüche 1 bis 3, **dadurch gekennzeichnet, daß** sie mit einer Carbonsäure auf einen pH-Wert von 1,5 bis 2,5, insbesondere 1,7 bis 2,2 und besonders bevorzugt 1,9 adjustiert wird.

5. Wässrige Zusammensetzung nach Anspruch 4, **dadurch gekennzeichnet, daß** sie mit Zitronensäure auf den pH-Wert 1,9 eingestellt wird.

6. Verwendung einer wässrigen Zusammensetzung nach einem der Ansprüche 1 bis 5, als Desinfektionsmittel für Hände, Human- oder Säugetierkörperteile und Human- oder Säugetiergewebe.

7. Verwendung einer wässrigen Zusammensetzung nach einem der Ansprüche 1 bis 5, als Desinfektionsmittel für Oberflächen von Gegenständen, Räumen, Geräten und textilem Gewebe, ohne Einschränkung was die Größe, Form und Materialbeschaffenheit der Oberflächen anbelangt

8. Verfahren zur Herstellung einer wässrigen Zusammensetzung nach einem der Ansprüche 1 bis 5, umfassend folgende Verfahrensschritte:
a) Herstellung des Polysaccharidsäuretensidkomplexes indem das Polysaccharid A mit der Carbonsäure B vermengt, mit Wasser versetzt und bei 60-80°C bis zur Bildung eines klaren Gels gerührt und danach das nichtionische oder amphotere Tensid C) zugegeben und weiter mit Wasser verdünnt wird, und Zugabe von b) Reaktionsprodukt von amphoteren Tensiden mit einer Carbonsäure zu a) Polysaccharidsäuretensidkomplex und
c) Einstellung des pH-Wertes mit einer Lösung einer Carbonsäure, bevorzugt Zitronensäure, auf einen pH-Wert 1,5-2,5 insbesondere1,7-2,2, besonders bevorzugt 1,9.

9. Wässrige Zusammensetzung gemäß Ansprüche 2 bis 5 hergestellt gemäß dem Verfahren nach Anspruch 8.

10. Verfahren nach Anspruch 8, wobei a) Chitosansalicylsäurecocoamidopropylbetainkomplex und b) ((Carboxymethyl)-trimethylammonium-salicylat) ist.
